# EUROPEAN PATENT APPLICATION

(11) **EP 4 427 695 A1**
(43) Date of publication of application: **11.09.2024**
(21) Application number: 22889360.8
(22) Date of filing: 03.11.2022
(51) Int. Cl.: A61B 18/18

(54) **ABLATION SYSTEM**

(30) Priority: 03.11.2021 CN 202111293952
(71) Applicant: Merryspring Medical Technology (Zhejiang) Co, Ltd., Jiaxing, Zhejiang 314499 (CN)
(72) Inventor: YANG, Yong, Chengdu, Sichuan 610000 (CN)
(74) Representative: Berggren Oy
(86) International application number: PCT/CN2022/129489
(87) International publication number: WO 2023/078338

(57) **Abstract**

Disclosed in the present invention is an ablation system. The ablation system comprises: a radio frequency generation module, which is used for generating a radio frequency output; a pulse generation module, which is used for generating a pulse output; an output control module, which is used for choosing, according to a characteristic of an ablation region, between ablation output modes, namely, radio frequency output and pulse output; and an ablation consumable, which is used for performing output for the ablation region according to the ablation output mode, which is selected by the output control module. According to the ablation system of the present invention, radio frequency ablation technology can be combined with pulsed electric field ablation technology, such that different application requirements in a clinical surgical operation are better met.

## Description

### Cross-reference to Related Applications

The present application claims priority to Chinese Patent Application No. 202111293952.3, entitled "Ablation System", filed on November 3, 2021, the disclosure of which is incorporated herein by reference in its entirety.

### Field of the Invention

The present invention relates to an ablation system, and particularly to a novel ablation system which can switch between, or mix, radio frequency ablation and pulse ablation.

### Background

A common thermal ablation technology in existing tumor minimally invasive treatment means is a radio frequency ablation technology in which a high-power radio frequency current is generated to act on a lesion point, such that ions in target tissue move to generate heat by friction, and the tissue in a target region is damaged by thermal coagulation. The purpose of damaging tumor cells is achieved using the process.

A pulsed electric field ablation technology has a principle that cell membranes and even cell nuclei are irreversibly electroporated to achieve an apoptosis purpose. The tumor cell is irreversibly electroporated by applying energy of a pulsed electric field to the tumor cell, thereby achieving a treatment purpose.

The radio frequency ablation technology has a larger ablation range and can treat cells in a larger cancer region particularly when used in conjunction with a perfusion pump, but the radio frequency ablation technology does not have selectivity, and ablation is performed indiscriminately, which possibly damages blood vessels, nerves, or the like, at the same time.

The pulsed electric field ablation technology is non-thermal ablation, and can protect adjacent tissue effectively. However, the pulsed electric field ablation technology has a limited action range, and meanwhile, a cardiac synchronization mode is required to be added at a position close to the heart in the pulsed electric field ablation technology, so as to guarantee discharge in a refractory period, and prevent an adverse effect of the energy of the pulsed electric field on the heart. Meanwhile, output of the pulsed electric field is usually a rectangular pulse wave and contains rich frequency components, such that a stimulation effect on peripheral motor nerves is prone to be generated.

In view of defects of the radio frequency ablation technology and the pulsed electric field technology in clinical application, existing pulsed electric field ablation devices and radio frequency ablation devices on the market are not integrated, such that if a doctor is required to use the two means simultaneously for treatment, great inconvenience is caused.

Therefore, an implementation of a novel ablation system is proposed herein, which combines the radio frequency ablation technology and the pulsed electric field ablation technology to better meet different application requirements in a clinical surgery.

### Summary

The present invention provides a novel ablation system which can switch between or mix radio frequency ablation and pulse ablation. The system according to the present invention can combine a radio frequency ablation technology and a pulsed electric field ablation technology, and choose between ablation output modes of radio frequency output and pulse output according to a characteristic of an ablation region, such that different application requirements in a clinical surgery are better met.

According to an embodiment of the present invention, there is provided an ablation system. The ablation system according to the present invention at least includes: a radio frequency generation module configured to generate radio frequency output; a pulse generation module configured to generate pulse output; an output control module configured to choose, according to a characteristic of an ablation region, between ablation output modes, namely, the radio frequency output and the pulse output; and an ablation consumable configured to perform output for the ablation region according to the ablation output mode chosen by the output control module.

Preferably, in the ablation system according to the present invention, the output control module chooses one of the following ablation output modes: radio frequency only output; pulse only output; and pulse and radio frequency hybrid output.

Preferably, in the ablation system according to the present invention, the ablation output mode of the output control module is manually chosen by an operator.

Preferably, in the ablation system according to the present invention, the output control module automatically chooses the ablation output mode.

Preferably, in the ablation system according to the present invention, the characteristic of the ablation region is a pre-estimated characteristic of the ablation region.

Preferably, in the ablation system according to the present invention, the characteristic of the ablation region is a size of the ablation region.

Preferably, in the ablation system according to the present invention, the size of the ablation region is determined by a parameter of pulse ablation. Preferably, the parameter of the pulse ablation includes at least one of: a pulse amplitude, a pulse width, a number of pulse groups, and a number of pulses.

Preferably, in the ablation system according to the present invention, the size of the ablation region is determined by a parameter of radio frequency ablation. Preferably, the parameter of the radio frequency ablation includes at least one of: a power, a temperature, a time, and a perfusion volume.

Preferably, in the ablation system according to the present invention, the characteristic of the ablation region is a position of the ablation region.

Preferably, in the ablation system according to the present invention, the ablation consumable has a corresponding position sensor. The ablation system may further include: a position signal acquisition module configured to acquire position information of the ablation consumable according to the position sensor of the ablation consumable; and a navigation system configured to determine the position of the ablation region according to the position information of the ablation consumable acquired by the position signal acquisition module.

Preferably, in the ablation system according to the present invention, the characteristic of the ablation region is an electrical impedance of the ablation region.

Preferably, in the ablation system according to the present invention, the ablation system further includes an impedance detection module configured to detect the electrical impedance of the ablation region.

Preferably, the electrical impedance is a complex impedance.

Preferably, in the ablation system according to the present invention, the characteristic of the ablation region is a PH level of the ablation region.

Preferably, in the ablation system according to the present invention, the characteristic of the ablation region is a change in a parameter of the ablation region before and after pre-test ablation.

Preferably, in the ablation system according to the present invention, the output control module may choose the ablation output mode according to at least two of the following characteristics of the ablation region: the size of the ablation region; the position of the ablation region; the electrical impedance of the ablation region; the PH level of the ablation region; the change in the parameter of the ablation region before and after pre-test ablation.

The output control module is a core of the system, and can perform output channel selection, electrical parameter setting and perfusion selection in addition to the selection of the energy output mode. The selection of the output mode is highlighted here, and pulse only output, radio frequency only output and pulse and radio frequency hybrid output can be realized. Specifically, the pulse and radio frequency hybrid output mentioned herein may be a pulse and radio frequency alternating output mode.

Different output modes can achieve different ablation effects. Thus, the operator may specifically choose a more suitable output mode for an ablation surgery depending on the ablation region, for example, on the characteristic of the ablation region. For example, the characteristic for characterizing the ablation region may include the size of the ablation region, the position of the ablation region, the electrical impedance of the ablation region, the PH level of the ablation region, and the change in the parameter of the ablation region before and after pre-test ablation. The operator can choose an ablation output mode in real time from the pulse only output, the radio frequency only output, and the pulse and radio frequency hybrid output according to at least one of the above characteristics of the ablation region.

When the hybrid output mode is chosen, surrounding tissue is not damaged indiscriminately, and a threshold of cells in the ablation region can be reduced to a certain degree; when the radio frequency only ablation mode is chosen, an influence on the heart can be prevented, and an ablation range is improved; when the pulsed only electric field ablation mode is chosen, the surrounding tissue can be better protected under the condition of ablating cancer cells through a selective performance thereof.

### Brief Description of the Drawings

The present invention will be more fully understood from the following detailed description in combination with the accompanying drawings in which like elements are numbered in a similar manner, and in the drawings:
FIG. 1 is a schematic block diagram of an ablation system according to an embodiment of the present invention.
FIG. 2 is a schematic diagram of a pulse generation circuit topology.
FIG. 3 is a schematic diagram of a pulse generation circuit topology which can realize sinusoidal pulses.
FIGs. 4A to 4E illustrate five pulse output waveforms.
FIG. 5 illustrates an output waveform realizing sinusoidal pulses.
FIG. 6 illustrates various parameters of a pulse output waveform.
FIG. 7 is a schematic diagram of a radio frequency generation circuit topology.
FIG. 8 is a schematic diagram of channel configuration by an output control module of the ablation system according to the embodiment of the present invention.
FIG. 9 is a schematic diagram of pulse and radio frequency alternating output.
FIGs. 10A to 10C are schematic diagrams of tissue ablation in three different example cases.

### Detailed Description

The technical solution of the present invention will be described in further detail below with reference to the embodiment and drawings, but the present invention is not limited to the following embodiment.

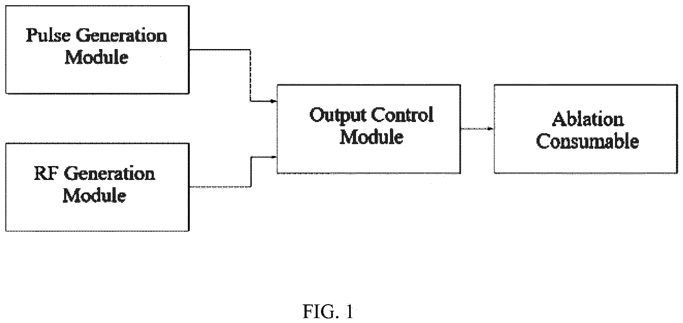

FIG. 1 is a schematic block diagram of an ablation system according to an embodiment of the present invention.

As shown in FIG. 1, main structural modules of the ablation system 100 according to the embodiment of the present invention include: a pulse generation module 101, a radio frequency (RF) generation module 102, an output control module 103, and an ablation consumable 104. It should be appreciated by those skilled in the art that the ablation system 100 includes other structural modules than the main structural modules listed above, which will be described in detail below. Since the technical solution of the present invention focuses on selection of an ablation energy output mode, the above modules are referred to herein as the main structural modules. However, it should be understood by those skilled in the art that in a practical ablation system, the main structural modules mentioned herein and other structural modules to be described below may be all constituent modules of the ablation system, play important roles, and may be combined together to realize an ablation surgery.

The pulse generation module 101 is configured to generate pulse output. Specifically, in a pulsed electric field ablation technology, cell membranes and even cell nuclei are irreversibly electroporated by the pulse output generated by the pulse generation module 101, thereby causing apoptosis. For example, in the tumor ablation surgery, tumor cells are irreversibly electroporated by applying energy of a pulsed electric field generated by the pulse generation module 101 to the tumor cells, thereby achieving a treatment purpose.

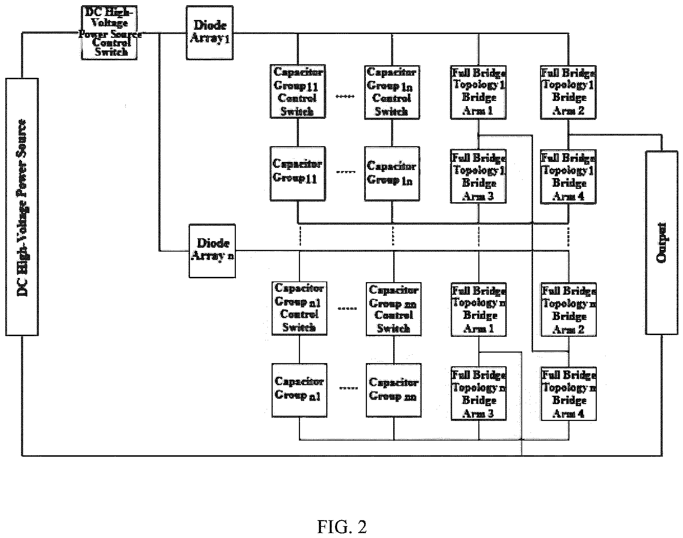

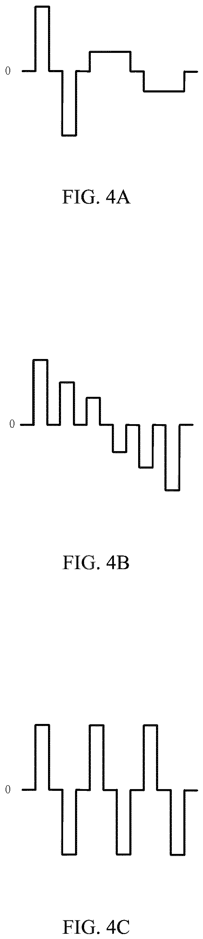

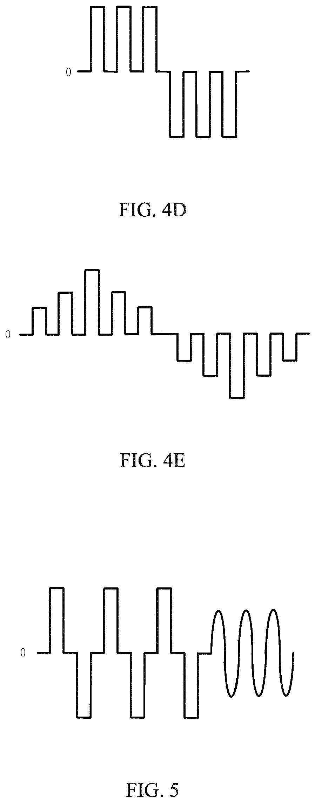

FIG. 2 is a schematic diagram of a pulse generation circuit topology as a preferred embodiment of the pulse generation module 101. The pulse circuit topology shown in FIG. 2 may realize any of the waveforms shown in FIGs. 4A to 4E and combinations thereof as described below.

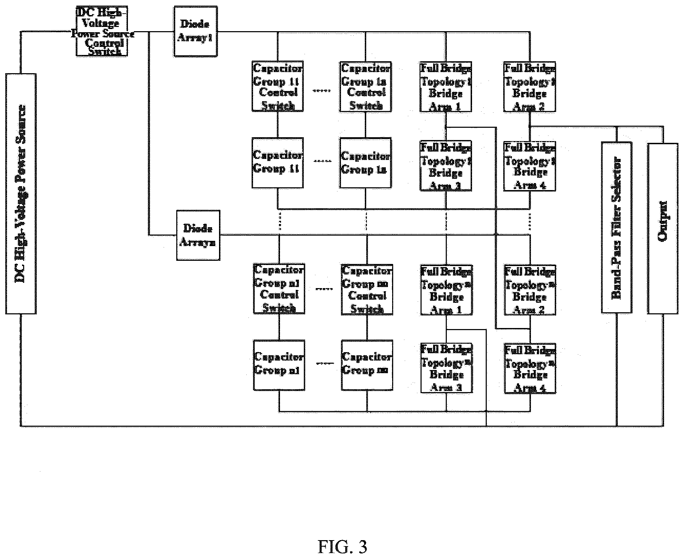

FIG. 3 is a schematic diagram of a pulse generation circuit topology which can realize sinusoidal pulses. As shown in FIG. 3, a band-pass filter is introduced in the pulse generation circuit topology to realize output of a sinusoidal pulse signal. The sinusoidal pulse signal can effectively solve electromagnetic interference of a pulsed electric field signal in a peripheral signal due to rich frequency components, and meanwhile can reduce a stimulation effect when the pulsed electric field acts.

FIGs. 4A to 4E illustrate five pulse output waveforms. FIG. 5 illustrates an output waveform realizing sinusoidal pulses. It should be understood by those skilled in the art that the pulse waveforms which can be actually output are not limited to the illustrated waveforms, and may be arbitrarily combined.

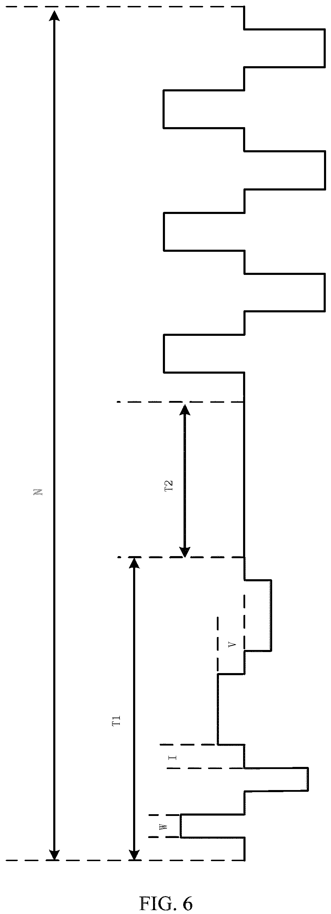

FIG. 6 illustrates various parameters of a pulse output waveform. As shown in FIG. 6, several common pulse output parameters are shown on a pulse schematic diagram. For example, common parameters of the pulsed electric field here are:
pulse amplitude V: ±300 ~ ±10kV;
pulse width W: 100ns ~ 100us;
pulse interval I: 0 ~ 1ms;
pulse group duration T1: less than 200ms;
pulse group interval T2: 10ms ~ 3s;
number of pulse groups N: 1 ~ 120.

The RF generation module 102 is configured to generate radio frequency output. Specifically, a high-power RF current is generated to act on a lesion point, such that ions in target tissue move to generate heat by friction, and the tissue in a target region is damaged by thermal coagulation. The purpose of damaging tumor cells is achieved using the process.

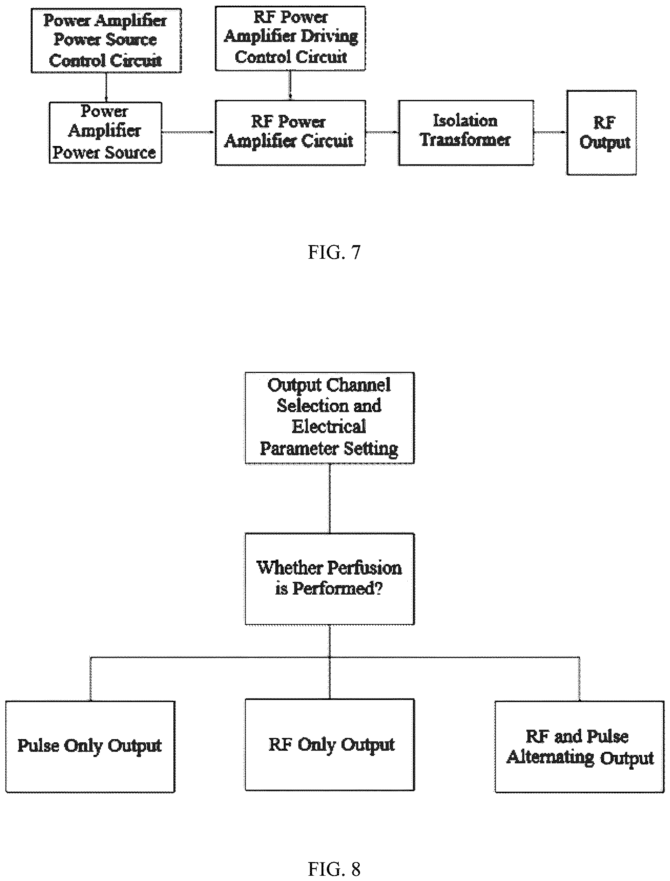

FIG. 7 is a schematic diagram of a RF generation circuit topology.

As shown in FIG. 7, the function of adjusting RF output energy is achieved by controlling a power amplifier power source control circuit to control energy output by a power amplifier power source. A functional power source and a RF power amplifier driving control circuit both act on a RF power amplifier circuit, and the RF output is obtained after an output signal is processed by an isolation transformer. Common output parameters are a maximum power of 300W and an output frequency of 30kHz ~ 2MHz.

The output control module 103 is configured to choose, according to a characteristic of an ablation region, between ablation output modes, namely, the radio frequency output and the pulse output. Specifically, the output control module may select one of the following ablation output modes: RF only output, pulse only output, and pulse and RF hybrid output.

The output control module 103 is a core constituent module of the ablation system 100 according to the present invention. In addition to the selection of the output mode, i.e., the realization of the pulse only output, the RF only output, and the pulse and RF hybrid output, the output control module 103 can perform output channel selection, electrical parameter setting, and perfusion selection. FIG. 8 is a schematic diagram of channel configuration by the output control module of the ablation system according to the embodiment of the present invention. As can be seen from FIG. 8, in one embodiment of the present invention, the output control module 103 first performs output channel selection and electrical parameter setting, and then judges whether perfusion is performed. After the above setting, selection and determination are finished, the output control module 103 may select an output mode of outputting only pulses, outputting only RFs, or outputting a mixture of pulses and RFs.

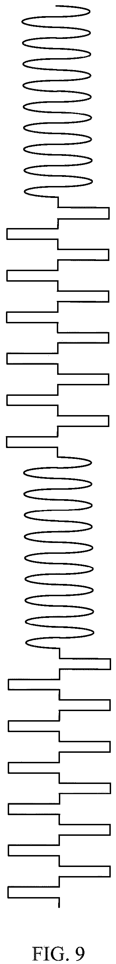

Specifically, the pulse and RF hybrid output mentioned herein may be a pulse and RF alternating output mode. FIG. 9 is a schematic diagram of pulse and RF alternating output.

The ablation consumable 104, such as an electrode for ablation, is configured to perform output for the ablation region according to the ablation output mode chosen by the output control module 103.

As mentioned above, in addition to the main structural modules listed above, the ablation system 100 includes other structural modules which may include: a pulse generation module, a RF generation module, a perfusion module, an impedance detection module, an electrical signal acquisition module, a self-detection module, a temperature detection module, a perception synchronization module, a human-machine interaction module, a position signal acquisition module, or the like (not shown in FIG. 1).

The perception synchronization module can receive an electrocardio synchronization signal, such that output can be performed in a refractory period of the heart when the pulsed electric field ablation technology is applied, and the energy of the pulsed electric field is prevented from influencing an electrical activity of the heart.

The impedance detection module can display a state of an output impedance in real time to learn whether the inserted consumable has a correct position, and meanwhile, the output can be stopped in time when the impedance is abnormal, so as to guarantee safety. A more detailed description is given below.

The electrical signal acquisition module can acquire signals, such as a voltage, a current, a power, energy, or the like, of the output energy of the pulsed electric field in real time and also can acquire signals, such as a voltage, a current, a power, energy, or the like, in the RF output process in real time, thus guaranteeing safety and effectiveness of the two energy output processes.

The self-detection module can detect an output circuit of the pulsed electric field and a RF output circuit before each output, and the output can be performed only after self detection, thus guaranteeing safety of the system.

The temperature detection module can detect a temperature condition of an output channel in real time. Temperature detection and control can be guaranteed during the RF output, and meanwhile, monitoring can be performed in a pulse output stage, thus preventing a heating condition caused by an overlong pulsed electric field output time. The temperature detection module also provides other channels for temperature detection of different parts, thus preventing an overtemperature condition caused by an action of the RF output on other parts.

The position signal acquisition module is mainly configured to acquire a magnetic signal of the ablation consumable 104 and then output the magnetic signal to facilitate a possible navigation system to process position information.

The human-machine interaction module is mainly configured to achieve a human-machine interaction function.

The perfusion module has two main functions: first, a temperature of the output channel can be reduced through perfusion, such that a better ablation depth is realized, and the function is particularly applied in the RF output mode; meanwhile, in the pulse output mode, when the ablation region is over large and a longer pulse ablation time is required, a temperature rise problem in a long-time pulse ablation process can be also solved by the perfusion function. Second, conductive liquid can be input through perfusion, such that the output channel is not suspended or the impedance is not over large, and the energy of the pulsed electric field and the RF can be guaranteed to reach certain load output conditions.

The following description goes back to the topic of output mode control.

As described above, the output control module 103 is configured to choose, according to the characteristic of the ablation region, between the ablation output modes, namely, the RF output and the pulse output.

The "selection" here may be manual selection of the ablation output mode by the operator, which is received by the output control module 103 and may be controlled accordingly. The output control module 103 may also automatically select or choose the ablation output mode by means of, for example, artificial intelligence.

The term "ablation region" here refers to a region to which ablation is directed, which may be, for example, tissue in an organism, such as a tumor. In some cases, the term "ablation region" and the term "ablation object" are used interchangeably and have the same meaning. The term "characteristic" here refers to a feature, attribute, nature and property of the ablation region, or another physical, mathematical and chemical variable or parameter which may be used to characterize the ablation region or ablation object. In some cases, the term "characteristic" and the following terms are used interchangeably and have the same meaning: feature, attribute, nature, property, variable, parameter, coordinate, or the like.

For example, as mentioned in the embodiments to be enumerated herein, the characteristic of the ablation region may include a size of the ablation region, a position of the ablation region, an electrical impedance of the ablation region, a PH level of the ablation region, a change in a parameter of the ablation region before and after pre-test ablation, or the like. The output control module 103 may select or choose the most appropriate ablation output mode based on one or any combination of the above characteristics.

The characteristic of the ablation region may be a pre-estimated characteristic of the ablation region. For example, the size of the ablation region and the position of the ablation region may be characteristics obtained by estimating the ablation region in advance based on existing parameters or settings.

A way in which the suitable output mode is selected or chosen depending on the characteristic of the ablation region is specifically described below.

In an embodiment, the characteristic of the ablation region is the size of the ablation region.

In the pulse ablation mode, for example, the size of the ablation region may be determined according to a parameter of the pulse ablation. The parameter of the pulse ablation includes at least one of: a pulse amplitude, a pulse width, a number of pulse groups, and a number of pulses. An ablation range here is used in combination with the matched consumable. If the matched consumable has 3 electrodes, parameter information of the electrodes can be determined by reading information of the matched consumable. Thus, a pulse ablation range is considered to be a spherical region formed by the 3 electrodes. The size of the spherical ablation region is related to the set pulse amplitude, pulse width, number of pulse groups and number of pulses. The pulse amplitude plays a decisive role and directly determines a magnitude of electric field intensity; the pulse width, the number of pulse groups and the number of pulses determine an action time, and play a factor enlarging or reducing role for the size of the ablation region. The size of the ablation region can be predicted by building a mathematical model of these parameters.

In the RF ablation mode, for example, the size of the ablation region may be determined according to a parameter of the RF ablation. The parameter of the RF ablation includes at least one of: a power, a temperature, a time, and a perfusion volume. Specifically, a range of the ablation region can be determined by a combination of the power, the temperature and the time, and meanwhile, if perfusion is used, the size of the ablation region can also be assessed in combination with perfusion flow as a parameter. The size of the ablation region can be predicted in combination with a mathematical model determined by these parameters.

In an embodiment, the characteristic of the ablation region is the position of the ablation region.

The ablation consumable may have a corresponding position sensor. The ablation system can obtain position information of the corresponding consumable by the position sensor, and the information is transmitted to the matched navigation system, such that the ablation position can be learnt. Specifically, as mentioned above, the ablation system 100 may further include the position signal acquisition module and the navigation system. The position signal acquisition module is configured to acquire the position information of the ablation consumable according to the position sensor of the ablation consumable. The navigation system is configured to determine the position of the ablation region according to the position information of the ablation consumable acquired by the position signal acquisition module. When a plurality of consumables are used simultaneously, the navigation system can estimate a position range of the tumor by using the position information. The navigation system transmits the information to the output control module of the ablation system, such that the output control module can be assisted in intelligently judging whether the selected ablation mode can meet requirements of the ablation region, and the most appropriate output mode can be appropriately selected to facilitate the electrode to perform output in a corresponding ablation energy mode.

In the above two embodiments, although the characteristics of the ablation region are the size and the position of the region respectively, the pulse and RF ablation range evaluation (i.e., the size) can be combined with the determination of the to-be-ablated region (i.e., the position) according to the position information, and the operator can be assisted in switching the channels in an intelligent mode to realize the pulse only output, the RF only output, and the RF and pulse alternating output. Furthermore, the perfusion can be intelligently switched at the same time. This is because the perfusion flow is related to the size of the ablation region. Thus, the requirements of the operator for the ablation region can be met. Meanwhile, the operator can manually choose the output mode, but ablation accuracy of the operator can be greatly improved by prompting the operator in the ablation region.

In an embodiment, the characteristic of the ablation region is the electrical impedance of the ablation region.

As previously mentioned, the ablation system 100 according to the present invention may include the impedance detection module configured to detect the electrical impedance of the ablation region.

In a preferred embodiment, the impedance detection module may detect a complex impedance. The complex impedance of the organism is an electrical impedance characteristic expressed by a biological organ, biological tissue and a biological cell under excitation of a safe current or voltage, and embodies a dielectric characteristic and a conductive characteristic of the tissue, the dielectric characteristic refers to a response of positive and negative bound charges of a biological cell dielectric medium to an external electric field, and the conductive characteristic refers to a response of conductive free charges (ions and electrons) in the biological cell to the external electric field.

A change of the cell during the ablation can be indirectly reflected by measuring the complex impedance. Therefore, sinusoidal signals covering 5kHz-200MHz are applied to the electrodes respectively, the applied signals are recovered and converted to obtain amplitude spectra and phase spectra reflecting different complex impedance characteristics under different frequencies of a frequency domain. Change conditions of biological tissue parameters and even ions can be learnt from the amplitude spectra and the phase spectra. After the pulse ablation or the RF ablation, corresponding ions, such as Na, K, protein, or the like, may change, and the complex impedance can visually reflect the change. When the parameters are changed to a certain degree, a change size and a change speed can indirectly reflect an ablation effect. Then, the operator can be assisted in knowing an ablation condition and judging whether the ablation mode can achieve an expected result. In this way, the operator can be intelligently assisted in dynamically switching the ablation modes to achieve the ablation effect.

Although in the preferred embodiment, detection of the complex impedance is taught, it should be appreciated by those skilled in the art that pure impedance detection is also contemplated herein, such that only determination of the impedance in a time domain is required to determine the effect. Certainly, it should be noted that richer data is obtained by using the pure impedance to judge the effect without using the complex impedance.

In an embodiment, the characteristic of the ablation region is the PH level of the ablation region.

That is, the ablation system 100 according to the present invention may further include a module for detecting the PH level of the ablation region. For example, by detecting a change of the PH value of the ablation region, the change of the ions can be indirectly learnt, and the ablation effect can be indirectly judged. In turn, depending on the change of the PH value, a more appropriate ablation output mode may be chosen.

In an embodiment, the characteristic of the ablation region is a change in a parameter of the ablation region before and after pre-test ablation.

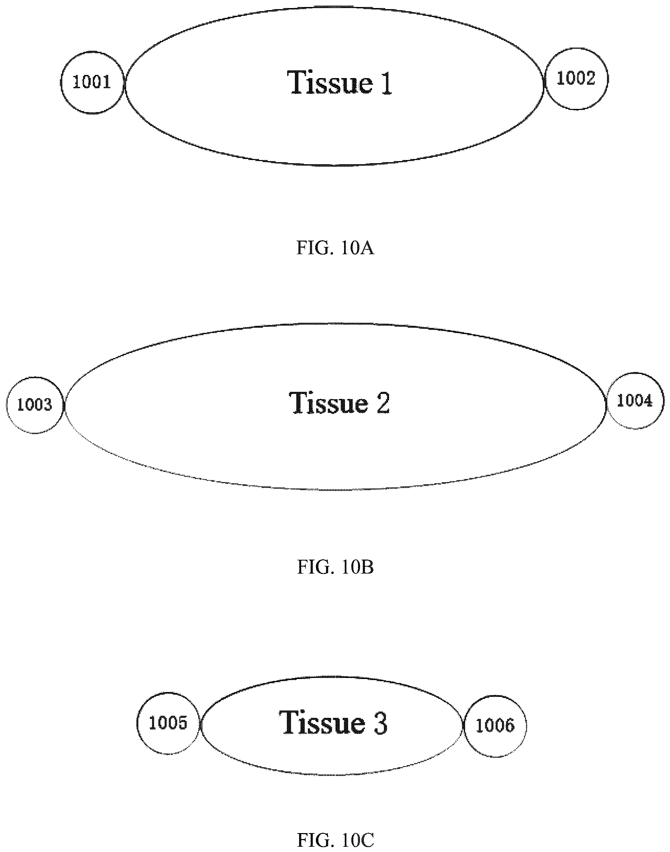

FIGs. 10A to 10C are schematic diagrams of tissue ablation in three different cases. Selection and effects of different modes of ablation will be described below by way of example in FIGs. 10A to 10C.

FIG. 10A is a schematic diagram of ablation of tissue in a first example case.

Tissue 1 between ablation points 1001, 1002 is ablated. At this point, tumor cells at the ablation points 1001, 1002 have a large difference, and the characteristic of the ablation region reflecting the difference includes the electrical impedance, the PH level, the change in the parameter before and after pre-test ablation, or the like. Depending on the characteristic of the ablation region, the pulse and RF hybrid ablation mode may be used. By controlling a certain radio frequency ablation temperature, surrounding tissue is not indiscriminately damaged, and meanwhile, a threshold of cells in the ablation region can be reduced to a certain degree, and at this point, the tumor cells in the ablation region can be better irreversibly electroporated by the pulse ablation mode.

FIG. 10B is a schematic diagram of ablation of tissue in a second example case.

Tissue 2 between ablation points 1003, 1004 is ablated. At this point, the tissue 2 between the ablation points 1003, 1004 is close to the heart, a tumor has a large area, and the reflecting characteristic of the ablation region may be the size, position, or the like, of the ablation region. Depending on the characteristic of the ablation region, only the RF ablation mode may be used to prevent the influence on the heart and increase the ablation range.

FIG. 10C is a schematic diagram of ablation of tissue in a third example case.

Tissue 3 between ablation points 1005, 1006 is ablated. At this point, the tissue 3 between the ablation points 1005, 1006 has abundant blood vessels, nerves, or bile ducts, and the reflecting characteristic of the ablation region may be the size, position, electrical impedance, PH level, change in the parameter before and after pre-test ablation, or the like. Depending on the characteristic of the ablation region, it is not suitable to use the RF ablation, it is more appropriate to use the pulsed electric field ablation alone, and the surrounding tissue is better protected in the case of ablating cancer cells through a selective performance thereof.

Although only three example cases are given here, it should be understood by those skilled in the art that the cases of the ablation region (ablation object) may vary widely. In any event, it is desirable to determine the appropriate ablation energy output based on the characteristic of the ablation region, such as the size, position, electrical impedance, PH level, change in the parameter before and after pre-test ablation, or the like, of the ablation region in accordance with the teachings of the present invention.

The embodiments of the present invention are not limited to the above-described embodiments, and various changes and modifications in form and detail may be made to the present invention by those skilled in the art without departing from the spirit and scope of the present invention, which are considered to fall within the protection scope of the present invention.

## Claims

1. An ablation system, comprising:
a radio frequency generation module configured to generate radio frequency output;
a pulse generation module configured to generate pulse output;
an output control module configured to choose, according to a characteristic of an ablation region, between ablation output modes of the radio frequency output and the pulse output; and
an ablation consumable configured to perform output for the ablation region according to the ablation output mode chosen by the output control module.

2. The ablation system according to claim 1, wherein the output control module is configured to select one of the following ablation output modes:
radio frequency only output;
pulse only output; and
pulse and radio frequency hybrid output.

3. The ablation system according to claim 1, wherein the output control module is configured that the ablation output mode of the output control module is manually chosen by an operator.

4. The ablation system according to claim 1, wherein the output control module is configured to automatically choose the ablation output mode.

5. The ablation system according to claim 1, wherein the characteristic of the ablation region is a pre-estimated characteristic of the ablation region.

6. The ablation system according to claim 1, wherein the characteristic of the ablation region is a size of the ablation region.

7. The ablation system according to claim 6, wherein the size of the ablation region is determined by a parameter of the pulse ablation.

8. The ablation system according to claim 7, wherein the parameter of the pulse ablation comprises at least one of: a pulse amplitude, a pulse width, a number of pulse groups, and a number of pulses.

9. The ablation system according to claim 6, wherein the size of the ablation region is determined by a parameter of the radio frequency ablation.

10. The ablation system according to claim 9, wherein the parameter of the radio frequency ablation comprises at least one of: a power, a temperature, a time, and a perfusion volume.

11. The ablation system according to claim 1, wherein the characteristic of the ablation region is a position of the ablation region.

12. The ablation system according to claim 11, wherein the ablation consumable has a corresponding position sensor, and
the ablation system further comprises:
a position signal acquisition module configured to acquire position information of the ablation consumable according to the position sensor of the ablation consumable; and
a navigation system configured to determine the position of the ablation region according to the position information of the ablation consumable acquired by the position signal acquisition module.

13. The ablation system according to claim 1, wherein the characteristic of the ablation region is an electrical impedance of the ablation region.

14. The ablation system according to claim 13, wherein the ablation system further comprises an impedance detection module configured to detect the electrical impedance of the ablation region.

15. The ablation system according to claim 14, wherein the electrical impedance is a complex impedance.

16. The ablation system according to claim 1, wherein the characteristic of the ablation region is a PH level of the ablation region.

17. The ablation system according to claim 1, wherein the characteristic of the ablation region is a change in a parameter of the ablation region before and after pre-test ablation.

18. The ablation system according to claim 1, wherein the output control module is further configured to choose the ablation output mode according to at least two of the following characteristics of the ablation region:
a size of the ablation region;
a position of the ablation region;
an electrical impedance of the ablation region;
a PH level of the ablation region; and
a change in the parameter of the ablation region before and after pre-test ablation.
